(19) Europäisches Patentamt
European Patent Office
Office européen des brevets

(11) Veröffentlichungsnummer: **0 216 299 A1**

(12) # EUROPÄISCHE PATENTANMELDUNG

(21) Anmeldenummer: 86112816.3

(22) Anmeldetag: 17.09.86

(51) Int. Cl.⁴: C 07 D 241/44
A 01 N 43/60, A 01 N 37/22

(30) Priorität: 21.09.85 DE 3533791

(43) Veröffentlichungstag der Anmeldung: 01.04.87 Patentblatt 87/14

(84) Benannte Vertragsstaaten: CH DE FR IT LI

(71) Anmelder: BASF Aktiengesellschaft
Carl-Bosch-Strasse 38
D-6700 Ludwigshafen(DE)

(72) Erfinder: Eicken, Karl, Dr.
Am Huettenwingert 12
D-6706 Wachenheim(DE)

(72) Erfinder: Spiegler, Wolfgang, Dr.
Westpreussenstrasse 5
D-6520 Worms 27(DE)

(72) Erfinder: Wuerzer, Bruno, Dr.
Ruedigerstrasse 13
D-6701 Otterstadt(DE)

(72) Erfinder: Meyer, Norbert, Dr.
Dossenheimer Weg 22
D.-6802 Ladenburg(DE)

(54) **Chinoxalinderivate, ein Verfahren zu ihrer Herstellung und ihre Verwendung zum Schutz von Kulturpflanzen vor der phytotoxischen Wirkung von Herbiziden.**

(57) Chinoxalinderivate der Formel

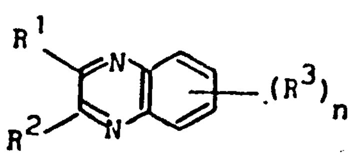

in der $R^1$, $R^2$, $R^3$ und n die in der Beschreibung genannte Bedeutung besitzen, ein Verfahren zu ihrer Herstellung, ihre Verwendung zum Schutz von Kulturpflanzen vor der phytotoxischen Wirkung von Herbiziden, herbizide Mittel, die Acetanilide der Formel

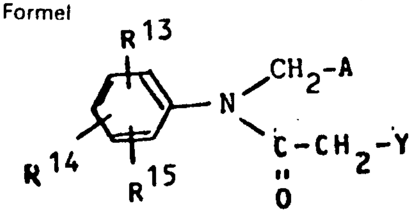

in der $R^{13}$, $R^{14}$, $R^{15}$, A und Y die in der Beschreibung genannte Bedeutung besitzen, als herbizide Wirkstoffe und Chinoxalinderivate als antidotische Verbindungen enthalten.

EP 0 216 299 A1

Chinoxalinderivate, ein Verfahren zu ihrer Herstellung und ihre Verwendung zum Schutz von Kulturpflanzen vor der phytotoxischen Wirkung von Herbiziden

Beschreibung

Die vorliegende Erfindung betrifft neue Chinoxalinderivate, ein Verfahren zu ihrer Herstellung, ihre Verwendung zum Schutz von Kulturpflanzen vor der phytotoxischen Wirkung von Herbiziden, neue herbizide Mittel, die Acetanilide als herbizid wirksame Stoffe und Chinoxalinderivate als antidotisch wirkende Verbindung enthalten, Verfahren zum Schutz von Kulturpflanzen vor der phytotoxischen Wirkung von Herbiziden auf Basis von Acetaniliden sowie Verfahren zur selektiven Bekämpfung von unerwünschtem Pflanzenwuchs mit den neuen herbiziden Mitteln.

Chinoxaline sind in der US-A-4 025 510, DE-A-2 942 364, EP-A-107 455 und DE-A-2 695 532 als Zwischenprodukte für Farbstoffe und Pharmazeutika oder als Pharmazeutika beschrieben.

Acetanilide der Formel IV

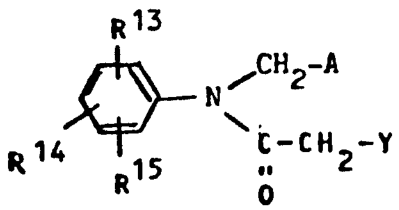

(IV),

in der

| | |
|---|---|
| $R^{13}$ | Wasserstoff, $C_1$-$C_5$-Alkyl oder $C_1$-$C_5$-Alkoxy, |
| $R^{14}$, $R^{15}$ | Wasserstoff, $C_1$-$C_5$-Alkyl, $C_1$-$C_5$-Alkoxy oder Halogen, |
| Y | Chlor oder Brom, und |
| A | $C_1$-$C_4$-Alkoxy, $C_2$-$C_8$-Alkoxyalkyl oder ein über einen Ringstickstoff gebundenes Azol, das gegebenenfalls durch Halogen, Phenyl, $C_1$-$C_4$-Alkyl, $C_1$-$C_4$-Alkoxy, $C_1$-$C_4$-Alkylthio, $C_1$-$C_4$-Perfluoralkyl, Cyano, Carboxyl oder $C_2$-$C_5$-Alkoxycarbonyl substituiert ist, wobei A auch für Salze der 2 oder 3 Stickstoffatome enthaltenden Azole stehen kann, |

bedeuten,

sind herbizid ausgezeichnet wirksam, führen aber bei Anwendung in Kulturen, beispielsweise in Mais, Reis, Kultursorghum oder Getreide zur Schädigung der Kulturpflanze (DE-A-2 648 008, DE-A-2 744 396, DE-A-2 305 495 und US-A-3 547 620).

Aufgabe der vorliegenden Erfindung war es, neue Chinoxalinderivate sowie neue antidotisch wirkende Verbindungen zu finden, die die Verträglichkeit der herbizid wirksamen Acetanilide der Formel IV gegenüber bestimmten Kulturpflanzen verbessern.

Es wurden Chinoxalinderivate der Formel I

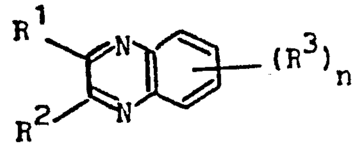

(I)

gefunden, in der

$R^1$ Halogen,

$R^2$ Wasserstoff, $C_1$-$C_4$-Alkyl, Halogen oder den Rest $COOR^4$, in dem $R^4$ für $C_1$-$C_6$-Alkyl oder $C_3$-$C_6$-Alkenyl steht, das jeweils unsubstituiert oder durch Halogen, Hydroxy, $C_1$-$C_6$-Alkoxy, $C_1$-$C_6$-Alkylthio, Amino, $C_1$-$C_4$-Monoalkylamino, $C_2$-$C_8$-Dialkylamino, gegebenenfalls durch Halogen, $C_1$-$C_4$-Alkyl oder $C_1$-$C_4$-Alkoxy substituiertes Phenyl, oder durch einen 5-gliedrigen Heterocyclus, der ein Sauerstoff- oder Schwefelatom enthält, substituiert ist,

$R^3$ den Rest $COOR^5$, in dem $R^5$ für Wasserstoff oder einen der Reste $R^4$ steht, oder

den Rest $CO\text{-}ON{=}C\langle{}^{R^6}_{R^7}$ , in dem $R^6$ und $R^7$ für $C_1$-$C_6$-Alkyl, $C_3$-$C_6$-Alkenyl, $C_3$-$C_6$-Cycloalkyl stehen oder in dem $R^6$ und $R^7$ zusammen für eine 4- bis 8-gliedrige Alkylenkette stehen, und

n eine ganze Zahl von 1 bis 4 bedeuten.

Ferner wurde gefunden, daß Chinoxalinderivate der Formel I a

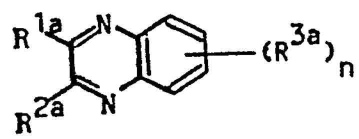

(Ia)

in der

$R^{1a}$ Halogen,

$R^{2a}$ Wasserstoff, $C_1$-$C_4$-Alkyl, Halogen oder den Rest $COOR^{4a}$, in dem $R^{4a}$ für $C_1$-$C_6$-Alkyl oder $C_3$-$C_6$-Alkenyl steht, das jeweils unsubstituiert oder durch Halogen, Hydroxy, $C_1$-$C_6$-Alkoxy, $C_1$-$C_6$-Alkylthio, Amino,

$C_1$-$C_4$-Monoalkylamino, $C_2$-$C_8$-Dialkylamino, gegebenenfalls durch Halogen, $C_1$-$C_4$-Alkyl oder $C_1$-$C_4$-Alkoxy substituiertes Phenyl, oder durch einen 5-gliedrigen Heterocyclus, der ein Sauerstoff- oder Schwefelatom enthält, substituiert ist,

$R^{3a}$ Wasserstoff, $C_1$-$C_6$-Alkyl, $C_3$-$C_6$-Cycloalkyl, $C_2$-$C_6$-Alkinyl, $C_1$-$C_6$-Halogenalkyl, $C_3$-$C_6$-Halogencycloalkyl, $C_2$-$C_6$-Halogenalkinyl, Halogen, Nitro, Cyano, den Rest $XR^{4a}$, in dem X für Sauerstoff, Schwefel oder Sulfonyl steht, den Rest $NR^{10a}R^{11a}$, in dem $R^{10a}$ und $R^{11a}$ für Wasserstoff, unsubstituiertes oder durch Hydroxy, Halogen, Phenyl oder durch einen 5-gliedrigen Heterocyclus, der ein Sauerstoff- oder Schwefelatom enthält, substituiertes $C_1$-$C_6$-Alkyl, für $C_3$-$C_6$-Alkenyl, $C_3$-$C_6$-Alkinyl, $C_3$-$C_6$-Cycloalkyl, unsubstituiertes oder durch Halogen, $C_1$-$C_4$-Alkyl oder $C_1$-$C_4$-Alkoxy substituiertes Phenyl stehen oder in dem $R^{10a}$ und $R^{11a}$ zusammen für eine 4- bis 6-gliedrige Alkylenkette, die gegebenenfalls durch ein Sauerstoffatom unterbrochen ist, stehen, den Rest $NH-CO-R^{12a}$, in dem $R^{12a}$ für gegebenenfalls durch Halogen substituiertes $C_1$-$C_4$-Alkyl oder für gegebenenfalls durch Halogen, $C_1$-$C_4$-Alkyl oder $C_1$-$C_4$-Alkoxy substituiertes Phenyl steht, den Rest $CO-R^{4a}$, den Rest $COOR^{5a}$, in dem $R^{5a}$ für Wasserstoff oder einen der Reste $R^{4a}$ steht,

den Rest $CO-ON=C(R^{6a})(R^{7a})$, in dem $R^{6a}$ und $R^{7a}$ für $C_1$-$C_6$-Alkyl, $C_3$-$C_6$-Alkenyl, $C_3$-$C_6$-Cycloalkyl stehen oder in dem $R^{6a}$ und $R^{7a}$ zusammen für eine 4- bis 8-gliedrige Alkylenkette stehen, die Reste $CO-NR^{10a}R^{11a}$ oder $SO_2-NR^{10a}R^{11a}$, durch die Reste $XR^{4a}$ oder $NR^{10a}R^{11a}$ substituiertes $C_1$-$C_6$-Alkyl oder $C_3$-$C_6$-Cycloalkyl oder durch den Rest $XR^{4a}$ substituiertes $C_2$-$C_6$-Alkinyl und

n eine Zahl von 1 bis 4 bedeuten,

sich in vorteilhafter Weise zur Steigerung der Kulturpflanzenverträglichkeit von herbiziden Acetaniliden der Formel IV eignen.

Unter den Verbindungen I sind diejenigen bevorzugt, in denen

$R^1$ - Halogen, besonders Chlor und Brom,

$R^2$ - Wasserstoff,
Halogen, besonders Fluor, Chlor und Brom,
den Rest $-COOR^4$,

$R^3$ - den Rest $-COOR^5$,
den Rest $-CO-ON=C(R^6)(R^7)$,

$R^4$ - $C_1$-$C_6$-Alkyl, besonders $C_1$-$C_4$-Alkyl wie Methyl, Ethyl, n-Propyl, iso-Propyl, n-Butyl, iso-Butyl, sec.-Butyl und tert.-Butyl,

- $C_3$-$C_6$-Alkenyl, besonders $C_3$-$C_5$-Alkenyl wie Prop-2-en-1-yl, But-2-en-1-yl, But-1-en-3-yl, 3-Methylbut-3-en-1-yl und 2-Methylbut-3-en-2-yl,

- $C_1$-$C_6$-Halogenalkyl, besonders $C_1$-$C_5$-Halogenalkyl wie Chlormethyl, Difluormethyl, Trifluormethyl, Pentafluorethyl, 2-Chlor-1,1,2,2--tetrafluorethyl, Nonafluorbutyl und 3,4-Dichlor-3-methylbutyl,

- $C_1$-$C_6$-Hydroxyalkyl, besonders $C_1$-$C_4$-Hydroxyalkyl wie 2-Hydroxypropyl, 3-Hydroxypropyl, 2-Hydroxybutyl, 3-Hydroxybutyl und 4-Hydroxybutyl,

- $C_2$-$C_{12}$-Alkoxyalkyl, besonders $C_2$-$C_8$-Alkoxyalkyl wie Methoxymethyl, 2-Methoxyethyl, 2-Ethoxyethyl, 1-Methoxyprop-2-yl, 2-Methoxypropyl, 2-Ethoxybutyl, 4-Methoxybutyl und 6-Methoxyhexyl,

- $C_2$-$C_{12}$-Alkylthioalkyl, besonders $C_2$-$C_8$-Alkylthioalkyl wie Methylthiomethyl, 2-Methylthioethyl, 2-Ethylthioethyl, 1-Methylthioprop-2-yl, 4-Methylthiobutyl und 6-Ethylthiohexyl,

- $C_1$-$C_6$-Aminoalkyl, besonders $C_1$-$C_4$-Aminoalkyl wie Aminomethyl, 2-Aminoethyl, 3-Aminopropyl, 1-Aminoprop-2-yl und 2-Aminobutyl,

- $C_2$-$C_{10}$-Monoalkylamino-alkyl, besonders $C_2$-$C_6$-Monoalkylamino--alkyl wie Methylaminomethyl und 2-(Methylamino)-ethyl,

- $C_3$-$C_{18}$-Dialkylamino-alkyl, besonders $C_3$-$C_{12}$-Dialkylamino-alkyl wie Dimethylamino-methyl, 2-(Dimethylamino)-ethyl, 3-(Dimethylamino)-ethyl, 3-(Dimethylamino)-propyl, 2-(Dimethylamino)-butyl, 2-(Diethylamino)-butyl, 2-(Dipropylamino)-butyl und 2-(Dibutylamino)-butyl,

- $C_7$-$C_{12}$-Phenylalkyl, besonders $C_7$-$C_{10}$-Phenylalkyl wie Benzyl, 2-Phenylethyl und 4-Phenylbutyl,

- $C_7$-$C_{12}$-Halogenphenyl-alkyl, besonders $C_7$-$C_{10}$-Halogenphenyl-alkyl wie p-Chlorbenzyl und p-Brombenzyl,

- $C_8$-$C_{16}$-Alkylphenyl-alkyl, besonders $C_8$-$C_{12}$-Alkylphenyl-alkyl wie p-Methylbenzyl,

- $C_8$-$C_{16}$-Alkoxyphenyl-alkyl, besonders $C_8$-$C_{12}$-Alkoxyphenyl-alkyl wie Methoxybenzyl,

- $C_1$-$C_6$-Alkyl durch einen 5-gliedrigen Heterocyclus substituiert, der ein Sauerstoff- oder Schwefelatom enthält, besonders $C_1$-$C_4$-Alkyl durch einen 5-gliedrigen Heterocyclus substituiert, der ein Sauerstoff- oder Schwefelatom enthält wie Furan-2-yl-methyl, Tetrahydrofuran-2-ylmethyl, Furan-2-ylethyl, Thien-2-yl-methyl, Tetrahydrothien-2-ylmethyl und Thien-3-ylethyl.

- $C_3$-$C_6$-Halogenalkenyl, besonders $C_3$-$C_6$-Chloralkenyl wie 1-Chlor-prop-1-en-3-yl, 1,3-Dichlorprop-1-en-3-yl und 3,4-Dichlor-2--methylbut-3-en-1-yl,

- $C_3$-$C_6$-Hydroxyalkenyl, besonders $C_3$-$C_5$-Hydroxyalkenyl wie 4-Hydroxybut-2-en-1-yl,

- $C_4$-$C_{12}$-Alkoxy-alkenyl, besonders $C_4$-$C_8$-Alkoxyalkenyl wie 4-Methoxybut-2-en-1-yl,

- $C_4$-$C_{12}$-Alkylthio-alkenyl, besonders $C_4$-$C_8$-Alkylthio-alkenyl wie 4-Methylthiobut-2-en-1-yl,

- $C_5$-$C_{14}$-Dialkylamino-alkenyl, besonders $C_5$-$C_{10}$-Dialkylamino--alkenyl wie 4-(Dimethylamino)but-2-en-1-yl,

- $C_9$-$C_{12}$-Phenylalkenyl wie 3-Phenylprop-2-en-1-yl,

- $C_9$-$C_{12}$-Halogenphenyl-alkenyl, besonders $C_9$-$C_{12}$-Chlorphenyl--alkenyl wie p-Chlorphenyl-prop-2-en-1-yl,

- $C_{10}$-$C_{16}$-Alkylphenyl-alkenyl, besonders $C_{10}$-$C_{14}$-Alkylphenyl--alkenyl wie p-Methylphenyl-prop-2-en-1-yl,

- $C_{10}$-$C_{16}$-Alkoxyphenyl-alkenyl, besonders $C_{10}$-$C_{14}$-Alkoxyphenyl--alkenyl wie p-Methoxyphenyl-prop-2-en-1-yl,

$R^5$ - die unter $R^4$ genannten Reste und

- Wasserstoff

$R^6$, $R^7$ - $C_1$-$C_6$-Alkyl, besonders $C_1$-$C_4$-Alkyl wie Methyl, Ethyl, n-Propyl, iso-Propyl, n-Butyl, iso-Butyl, sec.-Butyl und tert.-Butyl,

- $C_3$-$C_6$-Alkenyl, besonders $C_3$- und $C_4$-Alkenyl wie Prop-2-en-1-yl, But-2-en-1-yl und But-1-en-3-yl,

- $C_3$-$C_6$-Cycloalkyl, besonders $C_3$-, $C_5$- und $C_6$-Cycloalkyl wie Cyclopropyl, Cyclopentyl und Cyclohexyl,

- zusammen eine 4- bis 8-gliedrige Alkylenkette, besonders eine 4- bis 7-gliedrige Kette,

bedeuten und der Index

n - für eine ganze Zahl von 1 bis 4 steht.

Ganz besonders bevorzugte Chinoxalinderivate der Formel I sind solche, in denen $R^1$ Chlor, $R^2$ Chlor oder $COOR^4$, $R^3$ Wasserstoff, Methyl, Chlor, $COOR^4$ oder $CO{-}ON{=}C<^{R^6}_{R^7}$ und n die Zahlen 1 oder 2 bedeuten.

Unter den Verbindungen Ia sind diejenigen bevorzugt, in denen

$R^{1a}$ - Halogen, besonders Chlor und Brom,

$R^{2a}$ - Wasserstoff,

- Halogen, besonders Fluor, Chlor und Brom,

- den Rest $-COOR^4$,

$R^{3a}$ - Wasserstoff,

- $C_1$-$C_6$-Alkyl, besonders $C_1$-$C_4$-Alkyl wie Methyl, Ethyl, n-Propyl, iso-Propyl, n-Butyl, iso-Butyl, sec.-Butyl und tert.-Butyl,

- $C_3$-$C_6$-Cycloalkyl, besonders $C_3$-, $C_5$- und $C_6$-Cycloalkyl wie Cyclopropyl, Cyclopentyl und Cyclohexyl,

- $C_2$-$C_6$-Alkinyl, besonders $C_3$-$C_5$-Alkinyl wie Prop-2-in-1-yl, But-1-in-3-yl und 2-Methylbut-3-in-2-yl,

- $C_1$-$C_6$-Halogenalkyl, besonders $C_1$-$C_4$-Halogenalkyl wie Difluormethyl, Trifluormethyl, Pentafluorethyl, Nonafluorbutyl, Chlormethyl, Trichlormethyl und 2-Chlor-1,1,2,2-tetrafluorethyl,

- $C_3$-$C_6$-Halogencycloalkyl, besonders $C_3$-$C_6$-Fluor- und Chlorcycloalkyl,

- $C_2$-$C_6$-Halogenalkinyl, besonders $C_3$-$C_5$-Halogenalkinyl,

- Halogen, besonders Fluor, Chlor und Brom,

- den Rest $XR^{4a}$

- den Rest $COR^{4a}$

- den Rest $COOR^{5a}$

- den Rest $CO-ON=C\langle^{R^{6a}}_{R^{7a}}$

- den Rest $NR^{10a}R^{11a}$

- den Rest $CO-NR^{10a}R^{11a}$

- den Rest $SO_2-NR^{10a}R^{11a}$

- den Rest $NH-CO-R^{12a}$

- durch $XR^{4a}$ substituiertes $C_1$-$C_6$-Alkyl, besonders durch $XR^{4a}$ substituiertes $C_1$-$C_4$-Alkyl,

- durch $NR^{10a}R^{11a}$ substituiertes $C_1$-$C_6$-Alkyl, besonders durch $NR^{10a}R^{11a}$ substituiertes $C_1$-$C_4$-Alkyl,

- durch $XR^{4a}$ substituiertes $C_3$-$C_6$-Cycloalkyl, besonders durch $XR^{4a}$ substituiertes $C_3$-, $C_5$- und $C_6$-Cycloalkyl,

- durch $NR^{10a}R^{11a}$ substituiertes $C_3$-$C_6$-Cycloalkyl, besonders durch $NR^{10a}R^{11a}$ substituiertes $C_3$-, $C_5$- und $C_6$-Cycloalkyl,

- durch $XR^{4a}$ substituiertes $C_2$-$C_6$-Alkinyl, besonders durch $XR^{4a}$ substituiertes $C_3$-$C_5$-Alkinyl,

$R^{4a}$ - $C_1$-$C_6$-Alkyl, besonders $C_1$-$C_4$-Alkyl wie Methyl, Ethyl, n-Propyl, iso-Propyl, n-Butyl, iso-Butyl, sec.-Butyl und tert.-Butyl,

- $C_3$-$C_6$-Alkenyl, besonders $C_3$-$C_5$-Alkenyl wie Prop-2-en-1-yl, But-2-en-1-yl und But-1-en-3-yl, 3-Methylbut-3-en-1-yl und 2-Methylbut-3-en-2-yl,

- $C_1$-$C_6$-Halogenalkyl, besonders $C_1$-$C_5$-Halogenalkyl wie Chlormethyl, Difluormethyl, Trifluormethyl, Pentafluorethyl, 2-Chlor-1,1,2,2-tetrafluorethyl, Nonafluorbutyl und 3,4-Dichlor-3-methylbutyl,

- $C_1$-$C_6$-Hydroxyalkyl, besonders $C_1$-$C_4$-Hydroxyalkyl wie 2-Hydroxypropyl, 3-Hydroxypropyl, 2-Hydroxybutyl, 3-Hydroxybutyl und 4-Hydroxybutyl,

- $C_2$-$C_{12}$-Alkoxyalkyl, besonders $C_2$-$C_8$-Alkoxyalkyl wie Methoxymethyl, 2-Methoxyethyl, 2-Ethoxyethyl, 1-Methoxyprop-2-yl, 2-Methoxypropyl, 2-Ethoxybutyl, 4-Methoxybutyl und 6-Methoxyhexyl,

- $C_2$-$C_{12}$-Alkylthioalkyl, besonders $C_2$-$C_8$-Alkylthioalkyl wie Methylthiomethyl, 2-Methylthioethyl, 2-Ethylthioethyl, 1-Methylthioprop-2-yl, 4-Methylthiobutyl und 6-Ethylthiohexyl,

- $C_1$-$C_6$-Aminoalkyl, besonders $C_1$-$C_4$-Aminoalkyl wie Aminomethyl, 2-Aminoethyl, 3-Aminopropyl, 1-Aminoprop-2-yl und 2-Aminobutyl,

- $C_2$-$C_{10}$-Monoalkylamino-alkyl, besonders $C_2$-$C_6$-Monoalkylamino-alkyl wie Methylaminomethyl und 2-(Methylamino)-ethyl,

- $C_3$-$C_{18}$-Dialkylamino-alkyl, besonders $C_3$-$C_{12}$-Dialkylamino-alkyl wie Dimethylamino-methyl, 2-(Dimethylamino)-ethyl, 3-(Dimethylamino)-ethyl, 3-(Dimethylamino)-propyl, 2-(Dimethylamino)-butyl, 2-(Diethylamino)-butyl, 2-(Dipropylamino)-butyl und 2-(Dibutylamino)-butyl,

- $C_7$-$C_{12}$-Phenylalkyl, besonders $C_7$-$C_{10}$-Phenylalkyl wie Benzyl, 2-Phenylethyl und 4-Phenylbutyl,

- $C_7$-$C_{12}$-Halogenphenyl-alkyl, besonders $C_7$-$C_{10}$-Halogenphenyl-alkyl wie p-Chlorbenzyl und p-Brombenzyl,

- $C_8$-$C_{16}$-Alkylphenyl-alkyl, besonders $C_8$-$C_{12}$-Alkylphenyl-alkyl wie p-Methylbenzyl und p-tert.-Butylbenzyl,

- $C_8$-$C_{16}$-Alkoxyphenyl-alkyl, besonders $C_8$-$C_{12}$-Alkoxyphenyl-alkyl wie Methoxybenzyl,

- $C_1$-$C_6$-Alkyl durch einen 5-gliedrigen Heterocyclus substituiert, der ein Sauerstoff- oder Schwefelatom enthält, besonders

$C_1$-$C_4$-Alkyl durch einen 5-gliedrigen Heterocyclus substituiert, der ein Sauerstoff- oder Schwefelatom enthält wie Furan-2-ylmethyl, Tetrahydrofuran-2-ylmethyl, Furan-2-ylethyl, Thien-2-ylmethyl, Tetrahydrothien-2-ylmethyl und Thien-3-ylethyl.

- $C_3$-$C_6$-Halogenalkenyl, besonders $C_3$-$C_6$-Chloralkenyl wie 1-Chlorprop-1-en-3-yl, 1,3-Dichlorprop-1-en-3-yl und 3,4-Dichlor-2-methylbut-3-en-1-yl,

- $C_3$-$C_6$-Hydroxyalkenyl, besonders $C_3$-$C_5$-Hydroxyalkenyl wie 4-Hydroxybut-2-en-1-yl,

- $C_4$-$C_{12}$-Alkoxy-alkenyle, besonders $C_4$-$C_8$-Alkoxy-alkenyl wie 4-Methoxybut-2-en-1-yl,

- $C_4$-$C_{12}$-Alkylthio-alkenyl, besonders $C_4$-$C_8$-Alkylthio-alkenyl wie 4-Methylthiobut-2-en-1-yl,

- $C_5$-$C_{14}$-Dialkylamino-alkenyl, besonders $C_5$-$C_{10}$-Dialkylamino-alkenyl wie 4-(Dimethylamino)but-2-en-1-yl,

- $C_9$-$C_{12}$-Phenylalkenyl wie 3-Phenylprop-2-en-1-yl,

- $C_9$-$C_{12}$-Halogenphenyl-alkenyl, besonders $C_9$-$C_{12}$-Chlorphenyl-alkenyl wie p-Chlorphenyl-prop-2-en-1-yl,

- $C_{10}$-$C_{16}$-Alkylphenyl-alkenyl, besonders $C_{10}$-$C_{14}$-Alkylphenyl-alkenyl wie p-Methylphenyl-prop-2-en-1-yl,

- $C_{10}$-$C_{16}$-Alkoxyphenyl-alkenyl, besonders $C_{10}$-$C_{14}$-Alkoxyphenyl-alkenyl wie p-Methoxyphenyl-prop-2-en-1-yl,

$R^{5a}$ - die unter $R^{4a}$ genannten Reste und

- Wasserstoff

$R^{6a}$,
$R^{7a}$ - $C_1$-$C_6$-Alkyl, besonders $C_1$-$C_4$-Alkyl wie Methyl, Ethyl, n-Propyl, iso-Propyl, n-Butyl, iso-Butyl, sec.-Butyl und tert.-Butyl,

- $C_3$-$C_6$-Alkenyl, besonders $C_3$- und $C_4$-Alkenyl wie Prop-2-en-1-yl, But-2-en-1-yl und But-1-en-3-yl,

- $C_3$-$C_6$-Cycloalkyl, besonders $C_3$-, $C_5$- und $C_6$-Cycloalkyl wie Cyclopropyl, Cyclopentyl und Cyclohexyl,

- zusammen eine 4- bis 8-gliedrige Alkylenkette, besonders eine 4- bis 7-gliedrige Kette,

$R^{10a}$, $R^{11a}$ - Wasserstoff,

- $C_1$-$C_6$-Alkyl, besonders $C_1$-$C_4$-Alkyl wie Methyl, Ethyl, n-Propyl, iso-Propyl, n-Butyl, iso-Butyl, sec.-Butyl und tert.-Butyl,

- $C_1$-$C_6$-Hydroxyalkyl, besonders $C_1$-$C_4$-Hydroxyalkyl wie 2-Hydroxypropyl, 3-Hydroxypropyl, 2-Hydroxybutyl, 3-Hydroxybutyl und 4-Hydroxybutyl,

- $C_1$-$C_6$-Halogenalkyl, besonders $C_1$-$C_5$-Halogenalkyl wie Chlormethyl, Difluormethyl, Trifluormethyl, Pentafluorethyl, 2-Chlor-1,1,2,2-tetrafluorthyl, Nonafluorbutyl und 3,4-Dichlor--3-methylbutyl,

- $C_7$-$C_{12}$-Phenylalkyl, besonders $C_7$-$C_{10}$-Phenylalkyl wie Benzyl, 2-Phenylethyl und 4-Phenylbutyl,

- $C_1$-$C_6$-Alkyl durch einen 5-gliedrigen Heterocyclus substituiert, der ein Sauerstoff- oder Schwefelatom enthält, besonders $C_1$-$C_4$-Alkyl durch einen 5-gliedrigen Heterocyclus substituiert, der ein Sauerstoff- oder Schwefelatom enthält wie Furan-2-ylmethyl, Tetrahydrofuran-2-ylmethyl, Furan-2-ylethyl, Thien-2-ylmethyl, Tetrahydrothien-2-ylmethyl und Thien-3-ylethyl.

- $C_3$-$C_6$-Alkenyl, besonders $C_3$-$C_5$-Alkenyl wie Prop-2-en-1-yl, But-2-en-1-yl und But-1-en-3-yl, 3-Methylbut-3-en-1-yl und 2-Methylbut-3-en-2-yl,

- $C_3$-$C_6$-Halogenalkenyl, besonders $C_3$-$C_6$-Chloralkenyl wie 1-Chlorprop-1-en-3-yl, 1,3-Dichlorprop-1-en-3-yl und 3,4-Dichlor-2--methyl-but-3-en-1-yl,

- $C_3$-$C_6$-Hydroxyalkenyl, besonders $C_3$-$C_5$-Hydroxyalkenyl wie 4-Hydroxybut-2-en-1-yl,

- $C_9$-$C_{12}$-Phenylalkenyl wie 3-Phenylprop-2-en-1-yl,

- $C_3$-$C_6$-Alkenyl durch einen 5-gliedrigen Heterocyclus substituiert, der ein Sauerstoff- oder Schwefelatom enthält, besonders $C_3$-$C_5$-Alkenyl durch einen 5-gliedrigen Heterocyclus substituierten, der ein Sauerstoff- oder Schwefelatom enthält wie

1-(Furan-2-yl)-prop-1-en-3-yl, 4-(Tetrahydrofuran-2-yl)-2--methylbut-3-en-1-yl, 1-(Thien-2-yl)-prop-1-en-3-yl, 1-(Tetrahydrothien-2-yl)-prop-1-en-3-yl und 1-(Thien-3-yl)-prop-1-en-3-yl,

- $C_2$-$C_6$-Alkinyl, besonders $C_3$-$C_5$-Alkinyl wie Pro-2-in-1-yl, But-1-in-3-yl und 2-Methylbut-3-in-2-yl,

- $C_2$-$C_6$-Halogenalkinyl, besonders $C_3$-$C_5$-Halogenalkinyl,

- $C_8$-$C_{12}$-Phenylalkinyl wie 3-Phenylprop-2-in-1-yl,

- $C_2$-$C_6$-Alkinyl durch einen 5-gliedrigen Heterocyclus substituiert, der ein Sauerstoff- oder Schwefelatom enthält, besonders $C_3$-$C_5$-Alkinyl durch einen 5-gliedrigen Heterocyclus substituiert, der ein Sauerstoff- oder Schwefelatom enthält, wie 1-(Furan-2-yl)-but-1-in-3-yl, 1-(Tetrahydrofuran-2-yl)-but-1--in-3-yl, 1-(Thien-2-yl)-prop-1-en-3-yl, 1-(Tetrahydrothien-2--yl)-but-1-in-3-yl und 1-(Thien-3-yl)-but-1-in-3-yl,

- $C_3$-$C_6$-Cycloalkyl, besonders $C_3$-, $C_5$- und $C_6$-Cycloalkyl wie Cyclopropyl, Cyclopentyl und Cyclohexyl,

- $C_3$-$C_6$-Halogencycloalkyl, besonders $C_3$-$C_6$-Fluor- und Chlorcycloalkyl,

- $C_9$-$C_{12}$-Phenyl-cycloalkyl wie Phenyl-cyclopropyl, Phenylcyclopentyl und Phenyl-cyclohexyl,

- $C_3$-$C_6$-Cycloalkyl durch einen 5-gliedrigen Heterocyclus substituiert, der ein Sauerstoff- oder Schwefelatom enthält, besonders $C_3$-, $C_5$- und $C_6$-Cycloalkyl durch einen 5-gliedrigen Heterocyclus substituiert, der ein Sauerstoff- oder Schwefelatom enthält, wie 2-(Furan-2-yl)-cyclopropyl, 3-(Furan-2-yl)-cyclopentyl, 4-(Furan--2-yl)-cyclohexyl, 4-(Tetrahydrofuran-2-yl)-cyclohexyl, 4-(Thien--2-yl)-cyclohexyl, 4-(Tetrahydrothien-2-yl)-cyclohexyl und 4-(Thien-3-yl)-cyclohexyl,

- Phenyl,

- Halogenphenyl, besonders Fluor-, Chlor- und Bromphenyl wie 4-Fluorphenyl, 2,4-Difluorphenyl, 2,4,6-Trifluorphenyl, 4-Chlorphenyl, 2,4-Dichlorphenyl, 2,4,6-Trichlorphenyl und 4-Bromphenyl,

- $C_7$-$C_{10}$-Alkylphenyl wie 4-Methylphenyl, 4-Ethylphenyl, 4-tert.-Butylphenyl, 2,4-Dimethylphenyl und 2,4,6-Trimethylphenyl,

- $C_7$-$C_{10}$-Alkoxyphenyl wie 4-Methoxphenyl, 4-Ethoxyphenyl, 4-iso-Propoxyphenyl, 2,4-Dimethoxyphenyl und 2,4,6-Trimethoxyphenyl,

$R^{10a}$, $R^{11a}$- zusammen eine 4- bis 6-gliedrige Alkylenkette, die gegebenenfalls durch ein Sauerstoffatom unterbrochen ist, besonders eine 4- bis 5-gliedrige Alkylenkette, die gebenenfalls durch ein Sauerstoffatom unterbrochen ist wie $-(CH_2)_4-$, $-(CH_2)_5-$, und $-CH_2-CH_2-O-CH_2-CH_2-$,

$R^{12a}$- $C_1$-$C_4$-Alkyl wie Methyl, Ethyl, n-Propyl, iso-Propyl, n-Butyl, iso-Butyl, sec.-Butyl und tert.-Butyl.

- $C_1$-$C_5$-Halogenalkyl wie Chlormethyl, Difluormethyl, Trifluormethyl, Pentafluorethyl, 2-Chlor-1,1,2,2-tetrafluorethyl, Nonafluorbutyl und 3,4-Dichlor-3-methylbutyl,

- Phenyl

- Halogenphenyl, besonders Fluor-, Chlor- und Bromphenyl wie 4-Fluorphenyl, 2,4-Difluorphenyl, 2,4,6-Trifluorphenyl, 4-Chlorphenyl, 2,4-Dichlorphenyl, 2,4,6-Trichlorphenyl und 4-Bromphenyl,

- $C_7$-$C_{10}$-Alkylphenyl wie 4-Methylphenyl, 4-Ethylphenyl, 4-tert.-Butylphenyl, 2,4-Dimethylphenyl und 2,4,6-Trimethylphenyl,

- $C_7$-$C_{10}$-Alkoxyphenyl wie 4-Methoxyphenyl, 4-Ethoxyphenyl, 4-iso-Propoxyphenyl, 2,4-Dimethoxyphenyl und 2,4,6-Trimethoxyphenyl

bedeuten und der Index

n - für eine ganze Zahl von 1 bis 4 steht.

Besonders bevorzugte Chinoxalinderivate der Formel Ia sind solche, in denen $R^{1a}$ Chlor, $R^{2a}$ Chlor oder $COOR^{4a}$, $R^{3a}$ Wasserstoff, Methyl, Chlor, $COOR^{4a}$, $CO{-}O{-}N{=}C{<}^{R^{6a}}_{R^{7a}}$ oder $CO{-}N{<}^{R^{10a}}_{R^{11a}}$ und n die Zahlen 1 oder 2 bedeuten.

Die erfindungsgemäßen Chinoxalinderivate der Formel I erhält man vorteilhaft, indem man ein o-Phenylendiamin der Formel II

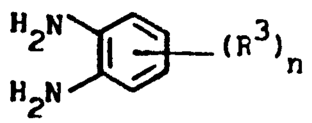
(II),

in der $R^3$ den Rest $COOR^5$, in dem $R^5$ für Wasserstoff oder einen der Reste $R^4$ steht oder den Rest $CO{-}ON{=}C{<}^{R^6}_{R^7}$, in dem $R^6$ und $R^7$ für $C_1$-$C_6$-Alkyl, $C_3$-$C_6$-Alkenyl, $C_3$-$C_6$-Cycloalkyl stehen oder in dem $R^6$ und $R^7$ zusammen für eine 4- bis 8-gliedrige Alkylenkette stehen und n eine ganze Zahl von 1 bis 4 bedeuten, mit einer Carbonylverbindung der Formel III

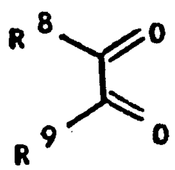
(III),

in der $R^8$ und $R^9$ Wasserstoff, $C_1$-$C_4$-Alkyl (z.B. Methyl, Ethyl, Propyl, Isopropyl oder Butyl), $C_1$-$C_4$-Alkoxy (z.B. Methoxy, Ethoxy, Propoxy, Isopropoxy oder Butoxy), $COOR^4$ bedeuten, mit der Maßgabe, daß zumindest einer der beiden Reste $R^8$ oder $R^9$ $C_1$-$C_4$-Alkoxy, bedeutet, bei einer Temperatur von 50 bis 300°C, gegebenenfalls in Anwesenheit eines inerten Verdünnungsmittels (z.B. Toluol, Chlorbenzol oder Xylol), zu einem entsprechenden Mono- oder Dihydroxychinoxalin umsetzt und anschließend mit einem üblichen Halogenierungsmittel (z.B. Phosphoroxychlorid, Phosphorpentachlorid sowie deren Mischungen oder Phosphortribromid) bei einer Temperatur von 50 bis 150°C, dessen Hydroxylgruppe(n) durch Halogen austauscht (vgl. dazu Weißberger Chem. of Heterocycl. Comp. Vol. 5, 1953, Seiten 242 ff. und Advances Het. Chem. 2, 203 (1963)).

Die folgenden Beispiele sollen die Erfindung näher erläutern.

Beispiel 1

18,5 g 6-Chlor-2,3-dihydroxychinoxalin, 70,5 g Phosphorpentachlorid und 95 ml Phosphorylchlorid wurden 4 Stunden am Rückfluß erhitzt. Nach dem Abkühlen wurde das Reaktionsgemisch auf Eis gegeben, abgesaugt, mit Eiswasser gewaschen und unter vermindertem Druck bei 50 °C getrocknet. Das Rohprodukt (19,9 g) ergab nach dem Auskochen mit 150 ml Toluol und Einengen des Filtrats 16,7 g 2,3,6-Trichlorchinoxalin. Fp.: 142°C (Verbindung Nr. 7).

Beispiel 2

Zu einer Lösung von 13,1 g 2,3-Dichlorchinoxalin-6-carbonsäurechlorid in 50 ml Toluol wurde bei -5 °C unter Rühren eine Mischung von 10,3 ml Benzylalkohol und 4,8 ml Pyridin zugetropft und bei 0°C 2 Stunden nachgerührt. Nach Zugabe von 100 ml Wasser und 100 ml Essigester wurde die organische Phase abgetrennt und das klare Filtrat unter vermindertem Druck eingeengt. Das Rohprodukt (16,4 g) lieferte nach Umkristallisieren aus Cyclohexan 10,8 g 2,3-Dichlorchinoxalin-6-carbonsäure-benzylester. Fp.: 116 - 117°C (Verbindung Nr. 61).

Analog einer der vorstehenden Methoden lassen sich die folgenden Verbindungen herstellen:

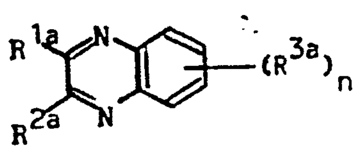

(Ia)

| Verb. Nr. | $R^{1a}$ | $R^{2a}$ | $(R^{3a})_n$ | Fp. [°C] |
|---|---|---|---|---|
| 1 | Cl | Cl | H | 150 |
| 2 | Cl | Cl | 5-F | |
| 3 | Cl | Cl | 6-F | 152 |
| 4 | Cl | Cl | 6,7-$F_2$ | 164 |
| 5 | Cl | Cl | 5,6,7,8-$F_4$ | 82 |
| 6 | Cl | Cl | 5-Cl | |

| Verb. Nr. | $R^{1a}$ | $R^{2a}$ | $(R^{3a})_n$ | Fp. [°C] |
|---|---|---|---|---|
| 7 | Cl | Cl | 6-Cl | 142 |
| 8 | Cl | Cl | 6,7-$Cl_2$ | 175 |
| 9 | Cl | Cl | 4,7-$Cl_2$ | |
| 10 | Cl | Cl | 4,8-$Cl_2$ | |
| 11 | Cl | Cl | 5,6,7,8-$Cl_4$ | 209 |
| 12 | Cl | Cl | 6-Br | 132 |
| 13 | Cl | Cl | 6-$CF_3$ | 83 |
| 14 | Cl | Cl | 6-$CCl_3$ | |
| 15 | Br | Br | H | 174 |
| 16 | Br | Br | 6-Cl | |
| 17 | Br | Br | 6,7-$Cl_2$ | |
| 18 | Cl | Cl | 5-$CH_3$ | 87 |
| 19 | Cl | Cl | 6-$CH_3$ | 108 |
| 20 | Cl | Cl | 6,7-$(CH_3)_2$ | 182 |
| 21 | Cl | Cl | 6-$OCH_3$ | 160 |
| 22 | Cl | Cl | 6,7-$(OCH_3)_2$ | |
| 23 | Cl | Cl | 4,8-$(OCH_3)_2$ | |
| 24 | Cl | Cl | 6-$NO_2$ | 154 |
| 25 | Cl | Cl | 6-Cl,8-$NO_2$ | |
| 26 | Cl | Cl | 4-Br,6-$NO_2$ | |
| 27 | Cl | Cl | 6-$SCH_3$ | |
| 28 | Cl | Cl | 6-$SO_2CH_3$ | |
| 29 | Cl | Cl | 4-$NH_2$ | |
| 30 | Cl | Cl | 6-$NH_2$ | |
| 31 | Cl | Cl | 6-NH-$COCH_3$ | |
| 32 | Cl | Cl | 6-NH-CO-$C_6H_5$ | |
| 33 | Cl | H | H | |
| 34 | Cl | H | 6-F | 132 |
| 35 | Cl | H | 5-Cl | 135 |
| 36 | Cl | H | 6-Cl | 47 |
| 37 | Cl | H | 7-Cl | |
| 38 | Cl | H | 5,7-$Cl_2$ | |
| 39 | Cl | H | 6,7-$Cl_2$ | |
| 40 | Cl | H | 5,6,7,8-$Cl_4$ | |
| 41 | Cl | H | 6-Br | 152 |
| 42 | Cl | H | 7-Br | |
| 43 | Cl | H | 6-J | |
| 44 | Cl | H | 6-$CF_3$ | 120 |
| 45 | Cl | H | 6-$CH_3$ | 104 |
| 46 | Cl | H | 6,7$(CH_3)_2$ | |
| 47 | Cl | H | 6-$OCH_3$ | |
| 48 | Cl | H | 5-$OCH_3$ | 113 |
| 49 | Cl | H | 6-$NO_2$ | |

| Verb. Nr. | $R^{1a}$ | $R^{2a}$ | $(R^{3a})_n$ | Fp. [°C] |
|---|---|---|---|---|
| 50 | Cl | H | $5-NO_2,7-Cl$ | |
| 51 | Cl | H | $6-SO_2CH_3$ | |
| 52 | Br | H | H | |
| 53 | Br | H | 6-Cl | |
| 54 | Cl | Cl | 6-CN | |
| 55 | Cl | Cl | $6-CO_2CH_3$ | 119 |
| 56 | Cl | Cl | $6-CO_2C_2H_5$ | |
| 57 | Cl | Cl | $6-CO_2-nC_4H_9$ | 46 |
| 58 | Cl | Cl | $6-CO_2-CH_2-CH{=}CH_2$ | 65 |
| 59 | Cl | Cl | $6-CO_2-CH_2-C{\equiv}CH$ | |
| 60 | Cl | Cl | $6-CO_2CH_2CH_2OC_2H_5$ | 52 |
| 61 | Cl | Cl | $6-CO_2-CH_2-C_6H_5$ | 117 |
| 62 | Cl | Cl | $6-CO_2-(CH_2)_2-C_6H_5$ | 109 |
| 63 | Cl | Cl | $6-CO_2-CH_2-(4-CH_3)-C_6H_4$ | 113 |
| 64 | Cl | Cl | $6-CO_2-CH_2-(4-Cl)C_6H_4$ | 122 |
| 65 | Cl | Cl | $6-CO_2-CH_2(2-Cl)C_6H_4$ | 121 |
| 66 | Cl | Cl | $6-CO_2-CH_2(4-CH_3O)C_6H_4$ | 107 |
| 67 | Cl | Cl | $6-CO_2-CH_2[4-(CH)_3C]C_6H_4$ | 129 |
| 68 | Cl | Cl | $6-CO_2-CH_2-CH_2Cl$ | 108 |
| 69 | Br | Br | $6-CO_2CH_3$ | |
| 70 | Br | Br | $6-CO_2CH_2-C_6H_5$ | |
| 71 | Cl | Cl | $6-CO-NH_2$ | 246 |
| 72 | Cl | Cl | $6-CO-NH-CH_3$ | 217 |
| 73 | Cl | Cl | $6-CO-NH-C_2H_5$ | 196 |
| 74 | Cl | Cl | $6-CO-NH-nC_4H_9$ | 152 |
| 75 | Cl | Cl | $6-CO-NH-CH_2-CH{=}CH_2$ | 167 |
| 76 | Cl | Cl | $6-CO-NH-c-C_3H_5$ | 209 |
| 77 | Cl | Cl | $6-CO-NH-c-C_6H_{11}$ | 209 |
| 78 | Cl | Cl | $6-CO-N(CH_3)_2$ | 178 |
| 79 | Cl | Cl | $6-CO-NH-CH_2CH_2OH$ | 183 |
| 80 | Cl | Cl | $6-CO-NHCH_2CH_2Cl$ | 166 |
| 81 | Cl | Cl | $6-CO-NH-CH_2-C_6H_5$ | 198 |
| 82 | Cl | Cl | $6-CO-NH(CH_2)_2C_6H_5$ | 172 |
| 83 | Cl | Cl | $6-CO-NHCH(CH_3)C_6H_5$ | 209 |
| 84 | Cl | Cl | $6-CO-NH-C_6H_5$ | 214 |
| 85 | Cl | Cl | $6-CO-NH-(4-CH_3)C_6H_4$ | |
| 86 | Cl | Cl | $6-CO-NH-(4-Cl)C_6H_4$ | |
| 87 | Cl | Cl | 6-CO- 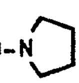 | |

| Verb. Nr. | $R^{1a}$ | $R^{2a}$ | $(R^{3a})_n$ | Fp. [°C] |
|---|---|---|---|---|
| 88 | Cl | Cl | 6-CO-N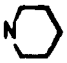 | 144 |
| 89 | Cl | Cl | 6-CO-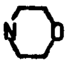 | 113 |
| 90 | Cl | Cl | $6-CO_2N=C(CH_3)_2$ | 174 |
| 91 | Cl | Cl | $6-CO_2N=$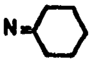 | 165 |
| 92 | Cl | Cl | $5-CO_2CH_3$ | |
| 93 | Cl | Cl | $5-CO_2CH_2-C_6H_5$ | |
| 94 | Cl | Cl | $5-CONH-CH_3$ | |
| 95 | Cl | Cl | $5-CO-NH-CH_2-C_6H_5$ | |
| 96 | Cl | Cl | $6-SO_2NH_2$ | |
| 97 | Cl | Cl | $6-SO_2NHCH_3$ | |
| 98 | Cl | Cl | $6-SO_2NH-CH_2-C_6H_5$ | |
| 99 | Cl | Cl | $6-SO_2NH-CH_2-CH_2-C_6H_5$ | |
| 100 | Cl | Cl | $6-SO_2NH-c-C_6H_{11}$ | |
| 101 | Cl | Cl | $6-SO_2N$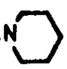 | |
| 102 | Cl | $CH_3$ | H | |
| 103 | Cl | $CH_3$ | 6-Cl | |
| 104 | Cl | $CH_3$ | $6,7-Cl_2$ | |
| 105 | Cl | $CO_2CH_3$ | H | |
| 106 | Cl | $CO_2CH_3$ | 6-Cl | |
| 107 | Cl | $CO_2CH_3$ | $6,7-Cl_2$ | |
| 108 | Cl | $CO_2CH_3$ | $6,7-(CH_3)_2$ | |
| 109 | Cl | $CO_2C_2H_5$ | H | |
| 110 | Cl | $CO_2C_2H_5$ | 6-Cl | |
| 111 | Cl | $CO_2C_2H_5$ | $6,7-Cl_2$ | 102 |
| 112 | Cl | $CO_2C_2H_5$ | $6,7-(CH_3)_2$ | 110 |
| 113 | Cl | $CO_2-CH_2-C_6H_5$ | $6,7-Cl_2$ | |
| 114 | Cl | $CO_2-CH_2-C_6H_5$ | $6,7-(CH_3)_2$ | |
| 115 | Cl | Cl | $6-CO_2-CH(CH_3)-C_6H_5$ | 117 |
| 116 | Cl | Cl | $6-CO_2-CH_2-$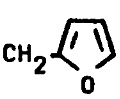 | 121 |
| 117 | Cl | Cl | $6-CO-NH-CH_2-$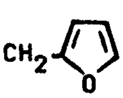 | 185 |

Acetanilide, deren Kulturpflanzenverträglichkeit durch die Chinoxalinderivate der Formel I a verbessert werden kann, sind solche der Formel IV, bei denen

$R^{13}$ für Wasserstoff, $C_1$-$C_5$-Alkyl wie Methyl, Ethyl, n-Propyl, i-Propyl, n-Butyl, sec-Butyl, i-Butyl, tert-Butyl oder n-Pentyl und verzweigte Pentylreste, $C_1$-$C_5$-Alkoxy wie Methoxy, Ethoxy, Propoxy, Butoxy oder Pentyloxy;

$R^{14}$ und $R^{15}$ für Wasserstoff, Halogen wie Fluor, Chlor, Brom oder Jod, $C_1$-$C_5$-Alkyl wie Methyl, Ethyl, n-Propyl, i-Propyl, n-Butyl, sec-Butyl, i-Butyl, tert-Butyl oder n-Pentyl und verzweigte Pentylreste, $C_1$-$C_5$-Alkoxy wie Methoxy, Ethoxy, Propoxy, Butoxy oder Pentyloxy;

Y für Chlor oder Brom und

A für $C_1$-$C_4$-Alkoxy oder $C_2$-$C_8$-Alkoxyalkyl, wie Methoxy, Ethoxy, Methoxymethyl, 2-Methoxyethyl, oder für ein über ein Ring-Stickstoffatom gebundenes Azol, wie Pyrrol, Pyrazol, Imidazol, 1,2,4-Triazol, 1,2,3-Triazol, Tetrazol, das gegebenenfalls einfach oder mehrfach durch Halogen, Phenyl, $C_1$-$C_4$-Alkyl, $C_1$-$C_4$-Alkoxy, $C_1$-$C_4$-Alkylthio, $C_1$-$C_4$-Perfluoralkyl, Cyano, Carboxyl oder $C_2$-$C_5$-Alkoxycarbonyl substituiert ist,

stehen.

Beispiele für substituierte Azole A sind 2,6-Dimethylpyrrol, Tetramethylpyrrol, 3(5)-Methylpyrazol, 4-Methylpyrazol, 3(5)-Ethylpyrazol, 4-Ethylpyrazol, 3(5)-Isopropylpyrazol, 4-Isopropylpyrazol, 3,5-Dimethylpyrazol, 3,4,5-Trimethylpyrazol, 3(5)-Phenylpyrazol, 4-Phenylpyrazol, 3,5-Diphenylpyrazol, 3(5)-Phenyl-5(3)-methylpyrazol, 3(5)-Chlorpyrazol, 4-Chlorpyrazol, 4-Brompyrazol, 3,5-Dimethyl-4-chlorpyrazol, 3,5-Dimethyl-4-brompyrazol, 4-Chlor-3(5)-methylpyrazol, 4-Methyl-3,5-dichlorpyrazol, 3(5)-Methyl-4,5(3)-dichlorpyrazol, 3(5)-Chlor-5(3)-methylpyrazol, 4-Methoxypyrazol, 3(5)-Methyl-5(3)-trifluormethylpyrazol, 3(5)-Methyl-5(3)-ethoxycarbonylpyrazol, 3(5)-Methyl-5(3)-methylthio-4-methoxy-carbonylpyrazol, 4-Cyanopyrazol, 4,5-Dichlor-imidazol, 2-Methyl-4,5-dichlorimidazol, 3(5)-Methyl-1,2,4-triazol, 3,5-Dimethyl-1,2,4-triazol, 3(5)-Chlor-1,2,4-triazol, 3,5-Dichlor-1,2,4-triazol, 4(5)-Methyl-1,2,3-triazol, 5-Methyltetrazol oder 5-Chlortetrazol.

Darüber hinaus kann der Rest A für den Fall, daß das Azol 2 oder 3 Stickstoffatome enthält, auch salzartig an eine der üblichen starken anorganischen oder organischen Säuren, wie Chlorwasserstoffsäure, Salpetersäure, Schwefelsäure, Trichloressigsäure, Methansulfonsäure, Perfluorhexansulfonsäure oder Dodecylbenzolsulfonsäure gebunden sein.

Bevorzugt sind Acetanilide der Formel IV, in der

$R^{13}$ Wasserstoff,
$R^{14}$,$R^{15}$ Methyl oder Ethyl in ortho-Position,
Y Chlor und
A Über einen Ringstickstoff gebundenes, gegebenenfalls methylsubstituiertes Pyrazol oder Triazol

bedeuten.

Als herbizide Chloracetanilide seien beispielsweise 2-Chlor-2',6'-dimethyl-N-(pyrazol-1-ylmethyl)acetanilid, 2-Chlor-2',6'-dimethyl-N-(3,5-dimethylpyrazol-1-ylmethyl)acetanilid, 2-Chlor-2',6'-diethyl-N-(pyrazol-1-ylmethyl)acetanilid, 2-Chlor-6'-ethyl-N-(pyrazol-1-ylmethyl)acet-o-toluidid, 2-Chlor-6'-ethyl-N-(3,5-dimethyl-pyrazol-1-ylmethyl)acet-o-toluidid, 2-Chlor-2',6'-dimethyl-N-(1,2,4-triazol-1-ylmethyl)acetanilid, 2-Chlor-6'-ethyl-N-(1,2,4-triazol-1-ylmethyl)acet-o-toluidid, 2-Chlor-2',6'-dithyl-N-(2"-propyloxyethyl)acetanilid, 2-Chlor-6'-ethyl-N-(2"-methoxy-1"-methylethyl)-acet-o-toluidid, 2-Chlor-2',6'-diethyl-N-(butoxymethyl)acetanilid, 2-Chlor-6'-ethyl-N-(ethoxymethyl)acet-o-toluidid, 2-Chlor-2',6'-dimethyl-N-(2"-methoxyethyl)acetanilid, 2-Chlor-6'-ethyl-N-(2"-butoxy-1"-methylethyl)acet-o-toluidid, 2-Chlor-2',6'-diethyl-N-(methoxymethyl)acetanilid oder 2-Chlor-2',6'-diethyl-N-(ethoxycarbonylmethyl)acetanilid genannt.

Herbizide Wirkstoffe und antidotisch wirkende Verbindungen können gemeinsam oder getrennt nach im Pflanzenbau üblichen Techniken für Pflanzenbehandlungsmittel ausgebracht werden. Dabei kann es sich um ein Einarbeiten in den Boden vor oder nach der Saat handeln.

Bei den Acetaniliden ist das gebräuchlichste Anwendungsverfahren die Ausbringung auf die Erdoberfläche unmittelbar nach Ablage der Samen oder in der Zeit zwischen Einsaat und Auflaufen der jungen Pflanzen. Auch eine Behandlung während des Auflaufens ist möglich. Von Bedeutung ist ferner die Anwendung im Nachauflaufverfahren in etablierten Kulturen. Dabei können sich die unerwünschten und zu bekämpfenden Pflanzenarten in unterschiedlichen Wuchsstadien befinden. Die Kulturpflanzen haben z.B. schon mehrere echte Blätter, während die unerwünschten Pflanzen gerade keimen oder auch schon Blätter haben.

Bei der getrennten Anwendung von antidotisch wirkender Verbindung und Herbizid sind folgende Anwendungsmethoden für das Antidot zu nennen:

Behandlung des Saatgutes der Kulturpflanzen als Trocken-, Naß-, Feucht- oder "Slurry"-Beizung sowie Eintauchen und Vorquellen des Saatgutes in Brühen, die eine antidotisch wirkende Verbindung enthalten, (seed

soaking). Die Applikation kann ferner während des Sähvorgangs in die Saatreihe erfolgen (in furrow-treatment). Weitere Anwendungstechniken entsprechen den an sich bekannten Verfahren bei Herbiziden.

Bei einer getrennten Ausbringung kann das Antidot vor oder nach dem Herbizid auf das Feld gebracht werden. Wichtig ist dabei, daß die antidotisch wirkende Verbindung der zu schützenden Kulturpflanze in dem Maße zur Verfügung steht, daß diese entweder von vornherein vor Schäden durch das Herbizid bewahrt wird oder sich mittels der antidotisch wirkenden Verbindung von einem bereits erlittenen Schaden erholen und weiterwachsen kann.

Antidot und herbizider Wirkstoff können hierbei als Spritzmittel in suspendierbarer, emulgierbarer oder löslicher Form oder als Granulate getrennt oder gemeinsam formuliert vorliegen.

Abhängig von den jeweils zu behandelnden Kulturen werden, bezogen auf ein bestimmtes Acetanilid, unterschiedliche Mengen einer antagonistisch wirkenden Verbindung benötigt. Die Mengenverhältnisse, in denen Acetanilid und Chinoxalinderivat eingesetzt werden können, sind über einen weiteren Bereich hinaus variabel. Das Gewichtsverhältnis zwischen herbizid wirksamem Acetanilid und antagonistisch wirkendem Chinoxalinderivat beträgt erfindungsgemäß 1 : 5 bis 1 : 0,01.

Die erfindungsgemäßen Chinoxalinderivate enthaltenden Mittel werden beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Tetrachlorkohlenstoff, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser, in Betracht.

Wäßrige Anwendungsformen können aus Emulsionskonzentraten, Pasten oder netzbaren Pulvern (Spritzpulvern) oder Öldispersionen durch Zusatz von

Wasser bereitet werden. Zur Herstellung von Emulsionen, Pasten oder Öldispersionen können herbizider Wirkstoff und/oder Antidot als solche oder in einem Öl als Lösungsmittel gelöst und mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen und Methylcellulose in Betracht.

Pulver-, Streu- und Stäubemittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Kalkstein, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe, und pflanzliche Produkte, wie Getreidemehle, Baumrinden-, Holz- und Nußschalenmehl, Cellulosepulver oder andere feste Trägerstoffe.

Die Formulierungen enthalten zwischen 0,1 und 95 Gewichtsprozent an herbizidem Wirkstoff und Antidot (gemeinsam oder jeweils einzeln formuliert), vorzugsweise zwischen 0,5 und 90 Gewichtsprozent. Die Aufwandmengen an herbizidem Wirkstoff betragen 0,02 bis 3 kg/ha. Diese Menge an herbizidem Wirkstoff wird gemeinsam oder getrennt mit einer solchen Menge

an Antidot ausgebracht, daß das Gewichtsverhältnis herbizider Wirkstoff : antagonistisch wirkender Verbindung 1 : 5 bis 1 : 0,01 beträgt.

Beispiele für Formulierungen sind:

I. 40 Gewichtsteile der Mischung aus vier Gewichtsteilen 2-Chlor-2',6'-dimethyl-N-(pyrazol-1-yl-methyl)-acetanilid und einem Gewichtsteil Verbindung Nr. 7 werden mit 10 Teilen Natriumsalz eines Phenolsulfonsäure-Harnstoff-Formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

II. 3 Gewichtsteile der Mischung aus einem Gewichtsteil 2-Chlor-2',6'-dimethyl-N-(pyrazol-1-yl-methyl)acetanilid und einem Gewichtsteil Verbindung Nr. 33 werden mit 97 Gewichtsteilen feinteiligem Kaolin innig vermischt. Man erhält auf diese Weise ein Stäubemittel, das 3 Gewichtsprozent des Wirkstoffs enthält.

III. 30 Gewichtsteile der Mischung aus einem Gewichtsteil 2-Chlor-2'-methyl-6'-ethyl-N-(1,2,4-triazol-1-yl-methyl)acetanilid und zwei Gewichtsteilen Verbindung Nr. 20 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 20 Teile der Mischung aus 8 Gewichtsteilen 2-Chlor-2'-methyl-,6'-ethyl-N-ethoxymethyl-acetanilid und einem Gewichtsteil Verbindung Nr. 31 werden mit 2 Teilen Calciumsalz der Dodecylbenzolsulfonsäure, 8 Teilen Fettalkohol-polyglykolether, 2 Teilen Natriumsalz eines Phenolsulfonsäure-Harnstoff-Formaldehyd- Kondensats und 68 Teilen eines paraffinischen Mineralöls innig vermischt. Man erhält eine stabile ölige Dispersion.

V. 20 Gewichtsteile der Mischung aus 10 Gewichtsteilen 2-Chlor-2',6'-dimethyl-N-(2-methoxyethyl)-acetanilid und einem Gewichtsteil Verbindung Nr. 61 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser

erhält man eine wäßrige Dispersion, die 0,02 Gewichtsprozent des Wirkstoffs enthält.

Die Wirkung der antidotisch wirkenden Verbindung zur Verbesserung der Kulturpflanzenverträglichkeit von herbiziden Wirkstoffen wird anhand nachfolgender Gewächshaus- und Freilandversuche belegt. Die Versuche zeigen, daß die Verträglichkeit der herbiziden Acetanilide mittels der Chinoxalinderivate entscheidend verbessert wird.

Als Herbizide wurden verwendet:

Herbizid A: 2-Chlor-2',6'-dimethyl-N-(3,5-dimethyl-pyrazol-1-ylmethyl)-acetanilid und

Herbizid B: 2-Chlor-2',6'-dimethyl-N-(pyrazol-1-ylmethyl)-acetanilid.

I) Saatgutbeizung mit Herbizidbehandlung im Vorauflaufverfahren

Die als Antidots geprüften Verbindungen wurden als Saatgutpuder formuliert. Samen von Reis (Oryza sativa) wurden mit Mengen von 10 und 50 g Aktivsubstanz, bezogen auf 100 kg Saatgut, eingestäubt. Man wog eine Menge von 50 g Samen ab, füllte diese in Plastikbeutel, gab die der Dosis erforderliche Aufwandmenge an Saatgutpuder hinzu und schüttelte den Inhalt sorgfältig durch. Einen Tag später säte man einen Teil der Samen in Plastikblumentöpfe von rund 300 $cm^3$ Inhalt. Als Boden diente lehmiger Sand mit annähernd 3 % Humus und pH 6,5 ($CaCl_2$).

Nach der Einsaat beregnete man die Töpfe leicht. Das Herbizid wurde in Wasser als Verteiler- und Trägermedium anschließend im Vorauflaufverfahren mittels feiner Düsen aufgespritzt. Die Aufwandmengen betrugen 0,05 und 0,1 kg Wirkstoff/ha.

Beim Einquellen des Saatgutes wurden von den antidotisch wirkenden Verbindungen Suspensionen von 10 und 1 000 ppm Wirkstoff in Wasser hergestellt. In dieser Brühe ließ man 50 g Reissaatgut für 24 Stunden quellen. Danach siebte man die Samen ab, säte sie in die oben bezeichneten Töpfe und führte die weitere Bearbeitung sowie die Behandlung mit Herbizid wie oben erwähnt durch. In einigen Fällen stellte man zunächst mit den antidotisch wirkenden Verbindungen eine 10%ige acetonische Lösung her und verdünnte diese mit Wasser (10 ml acetonische Lösung und 990 ml Wasser), bevor man die Samen einquoll.

II) Gemeinsames Ausbringen von antidotischen Verbindungen und herbiziden Wirkstoffen

Von der beispielhaft ausgewählten antidotisch wirkenden Verbindung Nr. 7 wurden 0,125 und 0,05 kg Wirkstoff/ha und vom Herbizid A 0,025 und 0,05 kg Wirkstoff/ha gemeinsam in Wasser als Träger- und Verteilermittel mit Hilfe von Spritzdüsen in einer für solche Applikationen geeigneten Spritzkabine im Vorauflaufverfahren aufgespritzt. Die Samen der Testpflanzen waren generell flach eingesät.

III) Getrennte Ausbringung von antidotischer Verbindung und herbiziden Wirkstoffen

a) Vorauflaufverfahren
Mit Reis besäte Töpfe wurden im Vorauflaufverfahren mit einer Aufwandmenge von 0,25 kg eines Chinoxalinderivates/ha behandelt. Die Töpfe wurden üblicherweise für die Keimung befeuchtet. Drei Tage nach dem Spritzen des Antidots erfolgte die Ausbringung des Herbizids. Die Reissamen begannen dann gerade zu keimen. Bei der als unerwünschten Beispielspflanze ausgesäten Hühnerhirse (Echinochloa crus-galli) war zeitweise das erste Blatt gebildet.

b) Nachauflaufverfahren
Analog ging man bei der Nachauflaufbehandlung (Blattbehandlung) vor. Man zog den Reis in den Versuchsgefäßen zunächst an, bis ungefähr das Ein- bis Zweiblattstadium erreicht war, dann bespritzte man ihn mit 0,25 kg eines Chinoxalinderivates/ha. Nach weiteren drei Tagen applizierte man das Herbizid in einer Dosis von 0,05 kg Wirkstoff/ha auf vorbehandelten Pflanzen.

Gewächshausversuche

Die Pflanzen wurden im Gewächshaus über 3 - 4 Wochen gepflegt und beobachtet. Die Bewertung der Einwirkung der Mittel erfolgte nach einer Skala von 0 bis 100. Hierbei bedeutet 0 normaler Aufgang und Entwicklung der Pflanzen, bezogen auf die unbehandelte Kontrolle, 100 entspricht einem völligen Ausbleiben der Keimung bzw. dem Absterben der Pflanzen.

Zur Überprüfung der Herbizidwirkung gegen unerwünschten Pflanzenwuchs wurde Echinochloa crus-galli (Hühnerhirse) in parallel stehende Töpfe eingesät und im Vorauflaufverfahren mit Antidot und Herbizid behandelt. Die weitere Behandlung erfolgte wie bei den Reispflanzen beschrieben.

Freilandversuche

Die Freilandversuche wurden auf einem Feld mit sandigem Lehm (Grobsand 58 %, Feinsand 21 %, Schluff 12 %, Ton 9 %, Humus 0,8 %; pH 6,2 ($CaCl_2$)) durchgeführt. Die Kulturpflanzen Sorghum bicolor (Kultursorghum) und Reis (Oryza sativa) säte man in Reihen in Kleinparzellen. Unkraut, wie z.B. Echinochloa crus-galli wuchs am Standort natürlich auf.

Die erfindungsgemäßen Mittel wurden folgendermaßen ausgebracht: Man nahm eine getrennte Applikation so vor, daß die antidotisch wirkende Verbindung in Aufwandmengen von 0,4 und 1,0 kg/ha zuerst nach der Aussaat im Vorauflaufverfahren ausgespritzt wurden. Das Herbizid applizierte man später in den Auflauf hinein bzw. zu einem frühen Nachauflauftermin auf die ersten Blätter in einer Aufwandmenge von 0,4 kg Wirkstoff/ha. Antidot und herbizider Wirkstoff wurden in Wasser als Träger- und Verteilermedium emulgiert oder suspendiert und mittels einer motorgetriebenen, auf einem Traktor montierten Parzellenspritze ausgebracht. Bei Mangel an natürlichen Niederschlägen wurde zusätzlich beregnet, um ein normales Auflaufen der Kulturen und Unkräuter zu gewährleisten.

Die Bewertung der Einwirkung der Mittel erfolgte ebenfalls nach der Skala von 0 bis 100. Die Versuche wurden über 4 und mehr Wochen nach dem Auflaufen beobachtet.

Ergebnisse im Gewächshaus

Im Gewächshaus wird am Beispiel der antagonistischen Verbindungen Nr. 7, 33, 77 und 78 gezeigt, daß diese bei Saatgutbeizung der Kulturpflanze Reis die unter den gegebenen Versuchsbedingungen erhebliche Phytotoxizität des Herbizids A deutlich reduzierten und somit das Herbizid verträglicher oder die Kulturpflanze widerstandsfähiger machen (siehe Tabelle 1).

Ebenso bewirken die Verbindungen Nr. 68, 75 und 79, beispielhaft als Saatbehandlungsmittel ausgebracht, eine erhebliche Verbesserung der Verträglichkeit des Herbizids A (siehe Tabelle 12).

Die beispielhaft ausgewählte antagonistische Verbindung Nr. 61 zeigte bei Anwendung des Herbizids B ebenfalls günstige Eigenschaften (siehe Tabelle 2).

Die beispielhaft ausgewählten Verbindungen Nr. 7, 20, 33, 39, 74, 90 und 91 schützten Reis vor der Phytotoxizität des Herbizids A, wenn das Reissaatgut vor dem Aussäen in den, die antidotisch wirkenden Verbindungen enthaltenden Brühen vorgequollen wurden. Die Herbizidbehandlung erfolgte

im Vorauflaufverfahren (siehe Tabellen 3 und 4). Desgleichen bot beispielsweise die Verbindung Nr. 1 nach dieser Methode Schutz für Reis gegen phytotoxische Einwirkungen des Herbizids (siehe Tabelle 5).

Im Vorauflaufverfahren bei gleichzeitiger Ausbringung von antidotischer Verbindung und herbizidem Wirkstoff reduzierte z.B. Verbindung Nr. 7 die Herbizidschäden an Reis, ohne die Herbizidwirkung gegen Echinochloa crus-galli zu beeinträchtigen (siehe Tabelle 6).

Bei der sukzessiven Ausbringung von antidotischen Verbindungen und Herbizid im Vorauflaufverfahren verringerten die beispielhaft ausgewählten Verbindungen Nr. 1 und 91 die Herbizidschäden. Die Wirkung gegen das Ungras Echinochloa crus-galli war hoch (siehe Tabelle 7).

Bei sukzessiver Ausbringung von antidotischen Verbindungen und Herbizid A im Nachauflaufverfahren auf die Blätter zeigten beispielsweise die Verbindungen Nr. 33, 61 und 74 günstige Wirkung (siehe Tabelle 8).

Ergebnisse im Freiland

In Freilandversuchen erwiesen sich beispielsweise die Verbindungen Nr. 33 und 61 als Schutzmittel für Kultursorghum gegen Schäden, verursacht durch das Herbizid A. Die Anwendung des Antidots erfolgte dabei im Vorauflaufverfahren nach der Saat, während das Herbizid 14 Tage später in den Auflauf der Kultur gespritzt wurde (siehe Tabelle 9).

In weiteren Versuchen, in denen das Antidot wiederum im Vorauflauf nach der Saat ausgebracht, das Herbizid aber erst nach dem Auflaufen auf die jungen Blätter gespritzt wurde, förderte der beispielhaft ausgewählte Verbindung Nr. 72 die Verträglichkeit von Kultursorghum gegenüber dem Herbizid A (siehe Tabelle 10).

In einem weiteren Versuch mit Reis als Kulturpflanze und analoger Anwendung schützte z.B. die Verbindung Nr. 91 Reis vor der Phytotoxizität des Herbizids A (siehe Tabelle 11).

Tabelle 1 - Anwendung von Antidots als Saatbeize und anschließende Anwendung von Herbizid A im Vorauflaufverfahren im Gewächshaus

| Antidotische Verbindung Nr. | Dosis [g/kg Saatgut] | Herbizid A [kg/ha] | Kulturpflanze Reis Schädigung [%] |
|---|---|---|---|
| - | - | 0,05 | 30 |
| | | 0,1 | 65 |
| 77 | 10 | 0,05 | 15 |
| | 50 | 0,05 | 10 |
| | 10 | 0,1 | 30 |
| | 50 | 0,1 | 20 |
| 33 | 10 | 0,05 | 10 |
| | 10 | 0,1 | 20 |
| | 50 | 0,1 | 20 |
| 78 | 10 | 0,05 | 0 |
| | 50 | 0,05 | 0 |
| | 10 | 0,1 | 20 |
| 7 | 50 | 0,05 | 0 |
| | 50 | 0,1 | 20 |

Tabelle 2 - Anwendung von Antidots als Saatbeize und anschließende Anwendung von Herbizid B im Vorauflaufverfahren im Gewächshaus

| Antidotische Verbindung Nr. | Dosis [g/kg Saatgut] | Herbizid B [kg/ha] | Kulturpflanze Reis Schädigung [%] |
|---|---|---|---|
| - | - | 0,05 | 65 |
| 61 | 10 | 0,05 | 20 |
| | 50 | 0,05 | 15 |

Tabelle 3 - Einquellen des Saatgutes in antidotische Verbindungen enthaltende Brühen und Vorauflaufanwendung von Herbizid A im Gewächshaus

| Antidotische Verbindung Nr. | Konzentration in der Tauchbrühe [ppm] | Herbizid A [kg/ha] | Kulturpflanze Reis Schädigung [%] |
|---|---|---|---|
| - | - | 0,05 | 55 |
| 33 | 10 | 0,05 | 0 |
| | 1000 | 0,05 | 10 |
| 7 | 10 | 0,05 | 22 |
| | 1000 | 0,05 | 10 |
| 20 | 10 | 0,05 | 10 |
| | 1000 | 0,05 | 10 |
| 91 | 10 | 0,05 | 20 |
| | 1000 | 0,05 | 25 |

Tabelle 4 - Einquellen von Saatgut in antidotische Verbindungen enthaltende Brühen und Vorauflaufanwendung von Herbizid A im Gewächshaus

| Antidotische Verbindung Nr. | Konzentration in der Tauchbrühe [ppm] | Herbizid A [kg/ha] | Kulturpflanze Reis Schädigung [%] |
|---|---|---|---|
| - | - | 0,05 | 55 |
| 90 | 1000 | 0,05 | 10 |
| 74 | 1000 | 0,05 | 25 |
| 39 | 1000 | 0,05 | 15 |

Tabelle 5 - Einquellen des Saatgutes in antidotische Verbindungen enthaltende Brühen, gefolgt von Vorauflaufanwendung des Herbizids A im Gewächshaus

| Antidotische Verbindung Nr. | Konzentration in der Tauchbrühe [ppm] | Herbizid A [kg/ha] | Kulturpflanze Reis Schädigung [%] |
|---|---|---|---|
| - | - | 0,1 | 65 |
| | | 0,05 | 40 |
| 1 | 10 | 0,1 | 20 |
| | 1000 | 0,1 | 10 |
| | 10 | 0,05 | 10 |
| | 1000 | 0,05 | 0 |

Tabelle 6 - Gleichzeitige Ausbringung von Mischungen aus Herbizid A und antidotischer Verbindung im Vorauflaufverfahren im Gewächshaus

| Antidotische Verbindung Nr. | Dosis [kg/ha] | Herbizid A [kg/ha] | Testpflanzen und Schädigung [%] Reis | Echinochloa crus-galli |
|---|---|---|---|---|
| - | - | 0,025 | 10 | 98 |
| | | 0,05 | 60 | 100 |
| 7 | 0,25 | 0,05 | 45 | 100 |
| | 0,125 | 0,025 | 0 | 98 |

Tabelle 7 - Getrennte Aufbringung von Antidot und Herbizid im Vorauflaufverfahren im Gewächshaus

| Antidotische Verbindung Nr. | Dosis [kg/ha] | Herbizid A [kg/ha] | Testpflanzen und Schädigung [%] Reis | Echinochloa crus-galli |
|---|---|---|---|---|
| - | - | 0,05 | 85 | 95 |
| 91 | 0,25 | 0,05 | 40 | 95 |
| 1 | 0,25 | 0,05 | 55 | 90 |

Tabelle 8 - Getrennte Aufbringung von Antidot und Herbizid im Nachauflaufverfahren im Gewächshaus

| Antidotische Verbindung Nr. | Dosis [kg/ha] | Herbizid A [kg/ha] | Kulturpflanze Reis Schädigung [%] |
|---|---|---|---|
| - | - | 0,05 | 32 |
| 33 | 0,25 | 0,05 | 20 |
| 74 | 0,25 | 0,05 | 10 |
| 61 | 0,25 | 0,05 | 20 |

Tabelle 9 - Anwendung von antidotischen Verbindungen im Vorauflaufverfahren nach der Saat, gefolgt von der Herbizidapplikation zur Zeit des Auflaufens der Kultur im Freiland (Intervall 14 Tage)

| Antidotische Verbindung Nr. | Dosis [kg/ha] | Herbizid A [kg/ha] | Testpflanze und Schädigung [%] Kultursorghum |
|---|---|---|---|
| - | - | 0,4 | 30 |
| 33 | 0,4 | 0,4 | 10 |
| | 1,0 | 0,4 | 10 |
| 61 | 0,4 | 0,4 | 10 |
| | 1,0 | 0,4 | 10 |

Tabelle 10 - Anwendung antidotischer Verbindungen im Vorauflaufverfahren nach der Saat, gefolgt von der Herbizidapplikation im Nachauflaufverfahren im Freiland

| Antidotische Verbindung Nr. | Dosis [kg/ha] | Herbizid A [kg/ha] | Testpflanzen und Schädigung [%] Kultursorghum |
|---|---|---|---|
| - | - | 0,4 | 20 |
| 72 | 0,4 | 0,4 | 5 |
| | 1,0 | 0,4 | 5 |

Tabelle 11 - Anwendung von antidotischer Verbindung im Vorauflaufverfahren nach der Saat, gefolgt von der Herbizidapplikation im Nachauflaufverfahren im Freiland

| Antidotische Verbindung Nr. | Dosis [kg/ha] | Herbizid A [kg/ha] | Testpflanzen und Schädigung [%] Reis | Echinochloa crus-galli |
|---|---|---|---|---|
| - | - | 0,4 | 20 | 75 |
| 91 | 0,4 | 0,4 | 5 | 70 |
| | 1,0 | 0,4 | 5 | 75 |

Tabelle 12 - Anwendung von Antidots als Saatbeize, gefolgt von Vorauflaufanwendung des Herbizids A im Gewächshaus

| Antidotische Verbindung Nr. | Dosis g a.S./kg Saatgut | Herbizid A kg/ha a.S. | Kulturpflanze Rei Schädigung % |
|---|---|---|---|
| - | - | 0,05 | 50 |
| 79 | 50 | 0,05 | 0 |
| | 10 | 0,05 | 0 |
| 75 | 50 | 0,05 | 10 |
| | 10 | 0,05 | 0 |
| 68 | 50 | 0,05 | 15 |

Patentansprüche

1. Chinoxalinderivate der Formel I

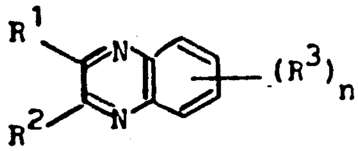

(I),

in der

$R^1$ Halogen,

$R^2$ Wasserstoff, $C_1$-$C_4$-Alkyl, Halogen oder den Rest $COOR^4$, in dem $R^4$ für $C_1$-$C_6$-Alkyl oder $C_3$-$C_6$-Alkenyl steht, das jeweils unsubstituiert oder durch Halogen, Hydroxy, $C_1$-$C_6$-Alkoxy, $C_1$-$C_6$-Alkylthio, Amino, $C_1$-$C_4$-Monoalkylamino, $C_2$-$C_8$-Dialkylamino, gegebenenfalls durch Halogen, $C_1$-$C_4$-Alkyl oder $C_1$-$C_4$-Alkoxy substituiertes Phenyl oder durch einen 5-gliedrigen Heterocyclus, der ein Sauerstoff- oder Schwefelatom enthält, substituiert ist,

$R^3$ den Rest $COOR^5$, in dem $R^5$ für Wasserstoff oder einen der Reste $R^4$ steht, oder

den Rest $CO{-}ON{=}C\genfrac{}{}{0pt}{}{R^6}{R^7}$, in dem $R^6$ und $R^7$ für $C_1$-$C_6$-Alkyl, $C_3$-$C_6$-Alkenyl, $C_3$-$C_6$-Cycloalkyl stehen oder in dem $R^6$ und $R^7$ zusammen für eine 4- bis 8-gliedrige Alkylenkette stehen, und

n eine ganze Zahl von 1 bis 4 bedeuten.

2. Verfahren zur Herstellung von Chinoxalinderivaten der Formel I gemäß Anspruch 1, dadurch gekennzeichnet, daß man ein o-Phenylendiamin der Formel II

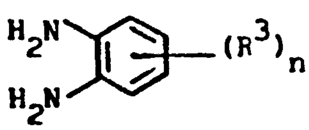

(II),

mit einer Carbonylverbindung der Formel III

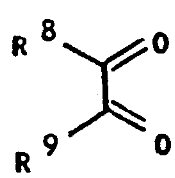

(III),

in der $R^8$ und $R^9$ Wasserstoff, $C_1$-$C_4$-Alkyl, $C_1$-$C_4$-Alkoxy, $COOR^4$ bedeuten, mit der Maßgabe, daß zumindest einer der beiden Reste $R^8$ oder $R^9$ $C_1$-$C_4$-Alkoxy bedeutet, bei einer Temperatur von 50 bis 300°C, gegebenenfalls in Anwesenheit eines inerten Verdünnungsmittels, zu einem entsprechenden Mono- oder Dihydroxychinoxalin umsetzt und anschließend mit einem Halogenierungsmittel, dessen Hydroxylgruppe(n) durch Halogen bei einer Temperatur von 50 bis 150°C austauscht.

3. Mittel zum Schutz von Kulturpflanzen vor der phytotoxischen Wirkung von Herbiziden auf Basis von Acetaniliden, enthaltend ein Chinoxalinderivat der Formel I a

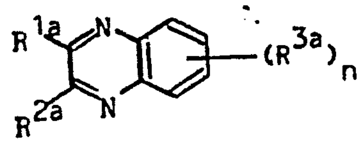

in der

$R^{1a}$ Halogen,

$R^{2a}$ Wasserstoff, $C_1$-$C_4$-Alkyl, Halogen oder den Rest $COOR^{4a}$, in dem $R^{4a}$ für $C_1$-$C_6$-Alkyl oder $C_3$-$C_6$-Alkenyl steht, das jeweils unsubstituiert oder durch Halogen, Hydroxy, $C_1$-$C_6$-Alkoxy, $C_1$-$C_6$-Alkylthio, Amino, $C_1$-$C_4$-Monoalkylamino, $C_2$-$C_8$-Dialkylamino, gegebenenfalls durch Halogen, $C_1$-$C_4$-Alkyl oder $C_1$-$C_4$-Alkoxy substituiertes Phenyl oder durch einen 5-gliedrigen Heterocyclus, der ein Sauerstoff- oder Schwefelatom enthält, substituiert ist,

$R^{3a}$ Wasserstoff, $C_1$-$C_6$-Alkyl, $C_3$-$C_6$-Cycloalkyl, $C_2$-$C_6$-Alkinyl, $C_1$-$C_6$-Halogenalkyl, $C_3$-$C_6$-Halogencycloalkyl, $C_2$-$C_6$-Halogenalkinyl, Halogen, Nitro, Cyano, den Rest $XR^{4a}$, in dem X für Sauerstoff, Schwefel oder Sulfonyl steht, den Rest $NR^{10a}R^{11a}$, in dem $R^{10a}$ und $R^{11a}$ für Wasserstoff, unsubstituiertes oder durch Hydroxy, Halogen, Phenyl oder durch einen 5-gliedrigen Heterocyclus, der ein Sauerstoff- oder Schwefelatom enthält, substituiertes $C_1$-$C_6$-Alkyl, für $C_3$-$C_6$-Alkenyl, $C_3$-$C_6$-Alkinyl, $C_3$-$C_6$-Cycloalkyl oder für unsubstituiertes oder durch Halogen, $C_1$-$C_4$-Alkyl oder $C_1$-$C_4$-Alkoxy substituiertes Phenyl stehen oder in dem $R^{10a}$ und $R^{11a}$ zusammen für eine 4- bis 6-gliedrige Alkylenkette, die gegebenenfalls durch ein Sauerstoffatom unter brochen ist, stehen, den Rest $NH$-$CO$-$R^{12a}$, in dem $R^{12a}$ für

gegebenenfalls durch Halogen substituiertes $C_1$-$C_4$-Alkyl oder für gegebenenfalls durch Halogen, $C_1$-$C_4$-Alkyl oder $C_1$-$C_4$-Alkoxy substituiertes Phenyl steht, den Rest CO-$R^{4a}$, den Rest $COOR^{5a}$, in dem $R^{5a}$ für Wasserstoff oder einen der Reste $R^{4a}$ steht,

den Rest 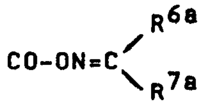 , in dem $R^{6a}$ und $R^{7a}$

für $C_1$-$C_6$-Alkyl, $C_3$-$C_6$-Alkenyl, $C_3$-$C_6$-Cycloalkyl stehen oder in dem $R^{6a}$ und $R^{7a}$ zusammen für eine 4- bis 8-gliedrige Alkylenkette stehen, die Reste CO-$NR^{10a}R^{11a}$ oder $SO_2$-$NR^{10a}R^{11a}$, durch die Reste $XR^{4a}$ oder $NR^{10a}R^{11a}$ substituiertes $C_1$-$C_6$-Alkyl oder $C_3$-$C_6$-Cycloalkyl, oder durch den Rest $XR^{4a}$ substituiertes $C_2$-$C_6$-Alkinyl, und

n eine Zahl von 1 bis 4 bedeuten.

4. Herbizides Mittel, gekennzeichnet durch einen Gehalt an mindestens einem Acetanilid der Formel IV

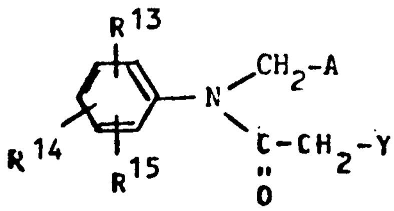 (IV),

in der

| | |
|---|---|
| $R^{13}$ | Wasserstoff, $C_1$-$C_5$-Alkyl oder $C_1$-$C_5$-Alkoxy, |
| $R^{14}$, $R^{15}$ | Wasserstoff, $C_1$-$C_5$-Alkyl, $C_1$-$C_5$-Alkoxy oder Halogen, |
| Y | Chlor oder Brom, und |
| A | $C_1$-$C_4$-Alkoxy, $C_2$-$C_8$-Alkoxyalkyl oder ein über einen Ringstickstoff gebundenes Azol, das gegebenenfalls durch Halogen, Phenyl, $C_1$-$C_4$-Alkyl, $C_1$-$C_4$-Alkoxy, $C_1$-$C_4$-Alkylthio, $C_1$-$C_4$-Perfluoralkyl, Cyano, Carboxyl oder $C_2$-$C_5$-Alkoxycarbonyl substituiert ist, wobei A auch für Salze der 2 oder 3 Stickstoffatome enthaltenden Azole stehen kann, |

bedeuten,
als herbizidem Wirkstoff und mindestens einem Chinoxalinderivat der Formel I a gemäß Anspruch 3 als antidotische Verbindung.

5. Herbizides Mittel gemäß Anspruch 4, dadurch gekennzeichnet, daß es als herbiziden Wirkstoff ein Acetanilid der Formel IV enthält, in der

| | |
|---|---|
| $R^{13}$ | Wasserstoff, |
| $R^{14}$, $R^{15}$ | Methyl oder Ethyl in ortho-Position, |
| Y | Chlor und |
| A | über einen Ringstickstoff gebundenes, gegebenenfalls methylsubstituiertes Pyrazol oder Triazol |

bedeuten.

6. Herbizides Mittel gemäß Anspruch 4, dadurch gekennzeichnet, daß das Gewichtsverhältnis Acetanilid : Chinoxalinderivat bei gemeinsamer oder getrennter Ausbringung 1 : 5 bis 1 : 0,01 beträgt.

7. Verfahren zum Schutz von Kulturpflanzen vor der phytotoxischen Wirkung von Herbiziden auf Basis von Acetaniliden, dadurch gekennzeichnet, daß man die Kulturpflanze, deren Standort oder deren Samen mit einer wirksamen Menge eines Chinoxalinderivats der Formel I a gemäß Anspruch 3 behandelt.

8. Verfahren zur selektiven Bekämpfung von unerwünschtem Pflanzenwuchs, dadurch gekennzeichnet, daß man Acetanilide der Formel IV gemäß Anspruch 4 und Chinoxalinderivate der Formel I a gemäß Anspruch 3 vor, während oder nach der Aussaat der Kulturpflanzen oder vor oder während des Auflaufens der Kulturpflanzen gleichzeitig oder nacheinander in beliebiger Reihenfolge ausbringt.

9. Verfahren zur selektiven Bekämpfung von unerwünschtem Pflanzenwuchs mit Acetaniliden der Formel IV gemäß Anspruch 4, dadurch gekennzeichnet, daß man das Saatgut der Kulturpflanzen mit einem Chinoxalinderivat der Formel I a gemäß Anspruch 3 behandelt.

10. Verfahren gemäß den Ansprüchen 7, 8 und 9, dadurch gekennzeichnet, daß die Kulturpflanzen Reis, Mais, Hirse oder Soja sind.

Europäisches Patentamt

0216299

## EUROPÄISCHER RECHERCHENBERICHT

Nummer der Anmeldung: EP 86112816.3

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US - A - 3 582 315 (SOPER)<br>* Zusammenfassung; Spalte 2, Zeilen 18-31; Spalte 4, Zeilen 33-39 * | 1,2,8-10 | C 07 D 241/44<br>A 01 N 43/60<br>A 01 N 37/22 |
| A | DE - A1 - 2 942 364 (BAYER)<br>* Formel I * | 1 | |
| A | CHEMICAL ABSTRACTS, Band 94, Nr. 19, 11. Mai 1981, Columbus, Ohio, USA<br>HOLLSTEIN, ULRICH; KRISOV, GALEN E. "Garbon-13 NMR studies on some 5-substituted quinoxalines" Seite 570, Spalte 1, Zusammenfassung Nr. 155 867 c<br>& Org. Magn. Reson. 1980, 14(4), 300-3 | 1 | RECHERCHIERTE SACHGEBIETE (Int Cl 4)<br>C 07 D 241/00<br>A 01 N |
| A | CHEMICAL ABSTRACTS, Band 101, Nr. 5, 30. Juli 1984, Columbus, Ohio, USA<br>ROUBINEK, FRANTISEK; BYDZOVSKY; VIKTOR; BUDESINSKY; Zdenek "Substituted 5- and 6-quinoxalinecarboxylic acids and their tuberculostatic activity" Seite 508, Spalte 2, Zusammenfassung Nr. 38 427 m<br>& Collect. Czech. Chem. Commun 1984, 49(1), 285-94 | 1 | |

Der vorliegende Recherchenbericht wurde für alle Patentansprüche erstellt.

| Recherchenort | Abschlußdatum der Recherche | Prüfer |
|---|---|---|
| WIEN | 02-11-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

**EUROPÄISCHER RECHERCHENBERICHT**

0216299
- 2 -
Nummer der Anmeldung

EP 86112816.3

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | CHEMICAL ABSTRACTS, Band 99, Nr. 3, 18. Juli 1983, Columbus, Ohio, USA<br>AHLBRECHT, HUBERTUS; DIETZ, MANFRED; RAAB, WERNER "Diels-Alder reactions with 1,4-dimethyl-2,3-dimethylenehexahydropyrazine; simple route to octahydro- and tetrahydroquinoxalines"<br>Seite 617, Spalte 2, Zusammenfassung Nr. 22 427 r<br>& Synthesis 1983, (3), 231-4<br>---- | 1 | |
| | | | RECHERCHIERTE SACHGEBIETE (Int Cl 4) |

Der vorliegende Recherchenbericht wurde für alle Patentansprüche erstellt

| Recherchenort | Abschlußdatum der Recherche | Prüfer |
|---|---|---|
| WIEN | 02-11-1986 | HAMMER |

KATEGORIE DER GENANNTEN DOKUMENTEN
X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E älteres Patentdokument, das jedoch erst am oder nach dem Anmeldedatum veröffentlicht worden ist
D in der Anmeldung angeführtes Dokument
L aus andern Gründen angeführtes Dokument

& Mitglied der gleichen Patentfamilie, übereinstimmendes Dokument

EPA Form 1503 03 82